# EUROPEAN PATENT SPECIFICATION

(11) **EP 2 488 629 B1**
(45) Date of publication and mention of the grant of the patent: **11.03.2020**
(21) Application number: 10768557.0
(22) Date of filing: 04.10.2010
(51) Int. Cl.: C12M 1/12, C12M 3/06, C12N 5/00, B01D 67/00

(54) **METHOD OF ASSEMBLING A HOLLOW FIBER BIOREACTOR**
VERFAHREN ZUR MONTAGE EINES HOHLFASERBIOREAKTORS
PROCÉDÉ D'ASSEMBLAGE D'UN BIORÉACTEUR À FIBRE CREUSE

(30) Priority: 12.10.2009 US 250758 P
(43) Date of publication of application: 22.08.2012
(73) Proprietor: Terumo BCT, Inc., Lakewood, CO 80215 (US)
(72) Inventor: DALBORG, Fredrik, B-3080 Tervuren (BE)
(74) Representative: J A Kemp LLP
(86) International application number: PCT/IB2010/002517
(87) International publication number: WO 2011/045644

(56) References cited:
- EP-A1- 1 795 254
- JP-A- 63 196 286
- JP-A- 2003 245 526
- US-A- 5 369 012
- HAUGEN ET AL.: "Effect of Different y-Irradiation Dosses on Cytotoxicity and Material Properties of Porous Polyether-Urethane Polymer", JOURNAL OF BIOMEDICAL MATERIALS RESEARCH. PART B: APPLIED BIOMATERIALS, vol. 80b, no. 2, February 2007 (2007-02), pages 415-423, XP002627714, DOI: 10.1002/jbmb

## Description

The instant invention relates to a method of treating a membrane surface before assembling such surfaces into a bioreactor.

The use of stem cells in a variety of medical treatments and therapies is receiving growing attention. Cell expansion systems can be used to grow stem cells, as well as other types of cells, such as bone marrow cells which may include stem cells. Stem cells which are expanded from donor cells can be used to repair or replace damaged or defective tissues and are considered for treating a wide range of diseases.

As an important component of a cell expansion system, a bioreactor, or cell growth chamber plays an important role in providing optimized environments for cell expansion. The bioreactor provides efficient nutrient supply to the cells and removal of metabolites, as well as furnishing a physiochemical environment conducive to cell growth.

Many types of bioreactors are currently available. Two of the most common include flat plate bioreactors and hollow fiber bioreactors. Flat plate bioreactors enable cells to grow on large flat surfaces, while hollow fiber bioreactors enable cells to grow on either the interior or exterior surface of the hollow fibers. The membranes are typically bundled together and enclosed within a casing or housing.

Many of the cells used to repair or replace damaged tissue are adherent cells. Adherent cells are defined in the context to the present invention as cells attaching to a substrate which are to be maintained, expanded, differentiated stored etc. Mesenchymal stem cells are adherent cells. Adherent cells, unlike suspension cells, can grow and divide only if they are attached to a surface.

Membranes which are commonly used to grow adherent cells may be made of naturally occurring material such as cellulose, or of man-made materials such as synthetic polymers. If the membranes are made of synthetic polymeric material, the surface of the material must be treated before cells are introduced into the system to enable the cells to adhere to the membrane.

Proteins such as fibronectin or those found in plasma are examples of naturally-occurring materials which are commonly used to coat the membranes. Gamma irradiation, electron beam graft polymerization and plasma treatment are examples of procedures used to chemically modify the membrane surface using radiation for cell adherance.

Membranes are typically treated with proteins or irradiated after the fibers have been enclosed in the housing. If radiation is used, the high doses of radiation required to modify the surface of the membranes may cause the unwanted side effect of breaking down other components of the bioreactor. Breakdown of the bioreactor housing may cause release of cytotoxic products into the cell growth area which may leach into the hollow fibers, inhibiting the growth of cells in the bioreactor. Current practice is to extensively rinse the bioreactor with various solutions to remove any toxic products before cells are placed in the bioreactor. This is done at the time of use, using several liters of electrolyte solutions in conjunction with an overnight exposure to a protein containing media.

PCT application PCT/EP2009/006847 (WO2010/034466) teaches use of a synthetic PES/PVP/PA membrane used to grow adherent cells wherein the membrane after production is subjected to beta or gamma ray or electron beam irradiation in a dose of from 12.5 to 175 kGy in the presence of oxygen. In this invention, the hollow fibers were assembled into a housing, potted, and the assembled bioreactor was gamma-ray irradiated as a final step before use. US 5369012 A describes treatment of a membrane for growing cells using flowing afterglow microwave discharge.

In actual use, the inventor of the present invention found that the gamma-irradiation process used to treat the synthetic membranes also damaged the housing. Irradiating the housing produced polymer breakdown products that were cytotoxic to the cells. Therefore, when using such a bioreactor, the cytotoxic products must be removed before cells are introduced into the bioreactor. This is done by washing the bioreactor multiple times before cell introduction.

Preventing the formation of cytotoxic products from the bioreactor as a result of irradiating the membrane would minimize the time and materials the end user spends decontaminating the bioreactor before use, as well as reducing the risk of contamination brought with the multiple rinsing steps and solutions. It is to the prevention of cytotoxic products contaminating the bioreactor that the present invention is directed.

It is an aim of the invention to prepare the surfaces of the polymeric membranes with gamma radiation and gas plasma treatment, while preventing the formation of cytotoxic products caused by irradiation of the polymeric housing of the cell growth module. This is accomplished by treating the membrane with gamma radiation and gas plasma before placing it in the polymeric casing.

The invention is defined in the claims.

The invention will be further described, by way of example, with reference to the drawings, in which:
Figure 1 illustrates an unassembled bundle of hollow fiber membranes to be used in a hollow fiber bioreactor.
Figure 2 is an external view of a polymeric housing including end caps of a hollow fiber bioreactor.
Figure 3 is a cross-sectional view of a cell growth module comprising a housing with the hollow fiber membrane inside.
Figure 4 illustrates a method of assembling a cell growth module without producing cytotoxins.

The membrane of the present invention can have various geometries. Although hollow fiber membranes are shown in the figures, the method of the present invention can be used with all forms of membranes, including flat sheet membranes. Hollow fiber membranes in cell growth systems have been shown to be favorable for cell expansion because the membranes in these systems allow for efficient supply of nutrients to the cells, removal of waste, and exchange of gases. Also, the hollow fibers provide a large surface area for cells to grow on in comparison to the volume of the membrane.

In the embodiments shown in the figures, cells may be grown inside the lumen or intracapillary space (IC space) of the hollow fibers, while cell growth media is flowed along the outside of the hollow fibers or extracapillary space (EC space). However, for the purposes of the invention, cells could also be grown in the EC space while the cell growth media is flowed in the IC space without departing from the scope of the invention.

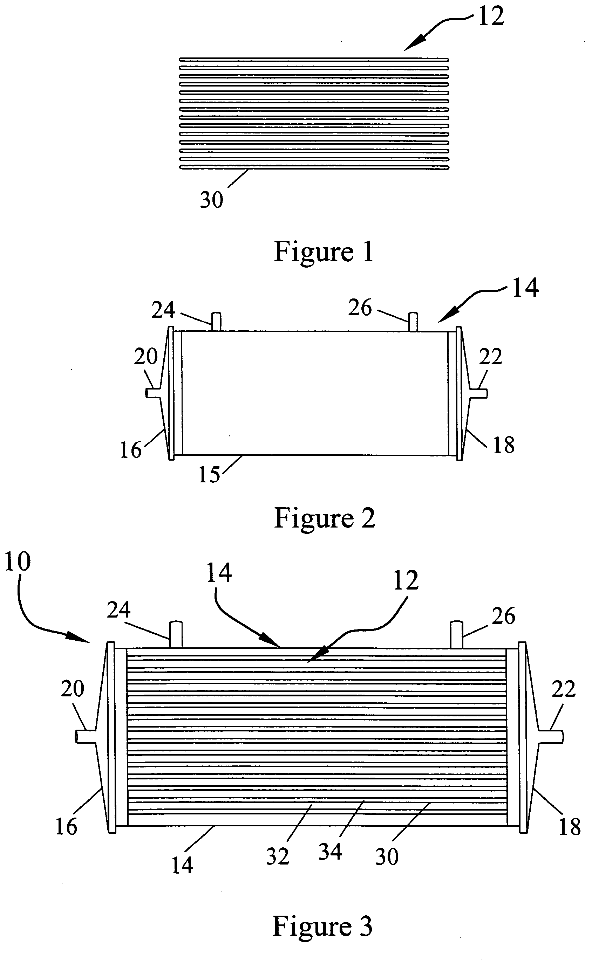

FIG. 1 illustrates a bundle of polymeric hollow fiber membranes **12** containing individual hollow fibers **30** which may be used in a bioreactor. An example of polymeric material which may be used to make the hollow fibers is described below. A plurality of hollow fibers are collectively referred to as a "membrane." The terms "bioreactor" and "cell growth chamber" are used interchangeably.

Any number of hollow fibers can be used in a cell growth chamber, provided the hollow fibers can be fluidly associated with the inlet and outlet ports of the cell growth chamber.

The hollow fibers may be made of a semi-permeable, biocompatible polymeric material. The semi-permeable membrane allows transfer of nutrients, waste and dissolved gases through the membrane between the EC space and IC space. In various embodiments, the molecular transfer characteristics of the hollow fiber membranes are chosen to minimize loss of expensive reagents necessary for cell growth such as growth factors, cytokines etc. from the cell growth side of the hollow fiber, while allowing metabolic waste products to diffuse through the membrane into the non-cellular side to be removed.

The hollow fibers **30** may be comprised of at least one hydrophobic polymer and at least one hydrophilic polymer. Suitable hydrophobic polymers include blends of polyethersulfone, polyamide, polyaramide, polyarylethersulfone, polysulfone, polyarylsulfone, polycarbonate, polyether, polyurethane, polyetherimode, and copolymers of those polymers. Suitable hydrophilic polymers include polyvinylpyrrolidone, polyethylene glycol, polyglycolmonoester, water soluble cellulosic derivates, polysorbate and polyethylene-polypropylene oxide copolymers.

In certain variations, the outer layer of each hollow fiber is characterized by a homogenous and open pore structure with a smooth or low surface roughness on the inner layer or cell adhesion side. The openings of the pores are in the size range of 0.5-3 um, and the number of pores on the outer surface of the fibers are in the range of 10,000 to 150,000 pores per mm². This outer layer has a thickness of about 1 to 10 um. The next layer in each hollow fiber is a second layer having the form of a sponge structure and, in a preferred embodiment of the present invention further embodiment, a thickness of about 1 to 15 um. This second layer serves as a support for the outer layer. A third layer next to the second layer has the form of finger-like structures. This third layer provides mechanical stability and a high void volume which gives the membrane a very low resistance to transporting molecules through the membrane. During use, the finger-like voids are filled with fluid and the fluid gives a lower resistance for diffusion and convection than a matrix with a sponge-filled structure having a lower void volume. This third layer has a thickness of 20 to 60 um.

Membranes such as those described above are commercially available from companies such as Gambro Dialysatoren GmbH (Hechingen, DE) and Membrana GmbH (Wuppertal, DE).

As discussed above, if adherent cells such as mesenchymal stem cells are to be expanded in a cell growth chamber, the polymeric fibers in such chamber must be surface modified to enhance cell growth and/or adherence of the cells to the membrane. Surface modification using naturally-occurring substances such as fibronectin, laminin, and collagen is commonly done, however, such surface treatments generally prevent the bioreactor from being reused. Therefore, surface modification using gamma-irradiation or plasma treatment to modify the membrane surface and allow membrane reuse is desirable.

The dose of gamma radiation used to treat the fibers ranges from 12.5 to 175 kGy. The radiation dose should be high enough to modify the surface of the membrane, but not so high as to disintegrate the membrane itself. Gamma irradiation can be administered using a Co-60 source. The membrane can be wet or dry when subjected to gamma irradiation, including from a complete absence of water to a total submersion in water. Although the amount of irradiation time necessary to apply the desired dose will vary with factors such as the strength of the source of irradiation, the membrane likely will require 6-7 hours of irradiation for a 25 kilogray dose and 17-20 hours for a 75 kilogray dose. Higher doses of radiation may be necessary for membranes that are saturated with water. The membrane can be gamma irradiated in unmodified air or in air with increased oxygen. After irradiation, the membrane surface is ready to be used for direct cell attachment and growth with no further treatment.

Plasma treatment, particularly gas plasma treatment, is additionally used to modify surfaces of membranes. Radio frequency glow discharge is one particular example of gas plasma treatment. To begin this type of gas plasma treatment, the membrane is placed into a vacuum chamber and gas is applied at a low pressure. An electromagnetic field or gas plasma is generated by subjecting the gas to an inductive or capacitive radio frequency electrical discharge. The gas absorbs energy from the electromagnetic field and ionizes, producing high-energy particles used to modify the surface of the membrane. The energized particles in a gas plasma include ions, electrons, radicals, and photons in the short wave ultraviolet light range, providing energy that transfers from the plasma to the membrane surface. Appropriate gases include inorganic gases such as helium, argon, and oxygen, and organic gases such as acetylene and pyridine, or a mix of both inorganic and organic gases. In general, power levels ranging between 10 watts and 3000 watts are acceptable, radio frequency frequency can range from 1 kHz to 100 MHz, with exposure times from 5 seconds to 12 hours, and gas pressures can range between 0.001 to 100 torr.

A combination of gamma radiation and gas plasma is used to treat the membrane.

FIG. 2 illustrates a housing or casing **14** into which a bundle of treated hollow fiber membranes **12** may be inserted. The housing **14** includes endcaps **16** and **18**, inlet ports **20** and **24**, outlet ports **22** and **26**, and a sleeve **15**.

Although cell growth chamber housing **14** is depicted as being cylindrical in shape, it can have any shape known in the art. Cell growth chamber housing **14** can be made of any type of biocompatible polymeric material known in the art such as polyvinylchloride or polycarbonate. The sleeve **15** and various component parts such as the end caps **16, 18** and ports **24, 26** are also typically made of biocompatible polymeric material.

FIG. 3 illustrates a cross-section of an assembled cell growth module **10.** The cell growth module **10** comprises a housing **14** and a bundle of hollow fiber membranes **12.** The hollow fiber membranes **12** are positioned inside the housing **14** of the cell growth module **10.**

In assembling the cell growth module **10,** the individual hollow fibers **30** are bundled together and adhered to the ends of the cell growth chamber by a connective material (also referred to herein as "potting" or "potting material"). The potting can be any suitable biocompatible material provided that the flow of media and cells from the inlet port into the hollow fibers is not obstructed and that the media and cells flow only into the hollow fibers. Exemplary potting material includes, but is not limited to, polyurethane. In various embodiments, the hollow fibers and potting may be cut through perpendicular to the central axis of the hollow fibers at each end to permit fluid flow into and out of the lumen of the fibers. End caps **16** and **18** are disposed at each end of the cell growth chamber.

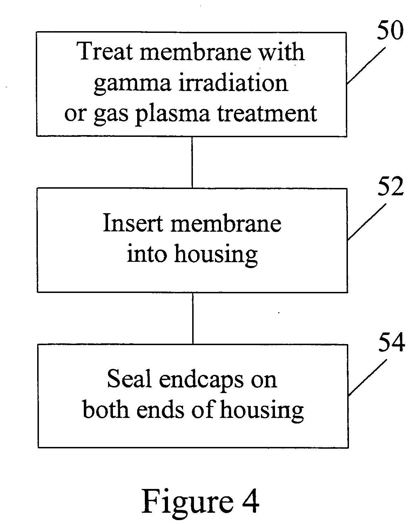

FIG. 4 illustrates a method for assembling a cell growth module **10** without producing cytotoxins due to exposing the housing **14** to electromagnetic radiation. First, the fibers are treated with gamma radiation and gas plasma treatment **50.** Next, the treated membrane **12** is inserted into the housing and potted **52.** Finally, end caps **16** and **18** are placed on each end of the sleeve **15** and sealed closed **54.** It should be noted that the fibers may be treated before or after forming them into a bundle for insertion into the housing.

No further surface treatment of the membrane **12** is necessary; thus the polymeric housing or casing **14** is not exposed to electromagnetic radiation, and thus the formation of cytotoxic products which inhibits cell growth is avoided.

## Claims

1. A method of assembling a cell growth module comprising a synthetic polymeric membrane and a synthetic polymeric housing, the method comprising:
treating the membrane with both gamma irradiation and gas plasma to modify the surface of the membrane to cause cells to adhere to the membrane; and
inserting the membrane into the housing, after the treating step to form the cell growth module.

2. The method of claim 1, wherein the membrane is a hollow fiber membrane.

3. The method of claim 2, wherein the hollow fiber membrane comprises a plurality of hollow fibers.

4. The method of claim 3, further comprising bundling the hollow fibers prior to the treating step.

5. The method of claim 3, further comprising bundling the hollow fibers after the treating step.

6. The method of any one of claims 3, 4 or 5, further comprising:
after the inserting step, adhering the hollow fibers to each end of the housing using a potting material to create a fluid-tight seal with the housing.

7. The method of claim 6, wherein the potting material comprises polyurethane.

8. The method of any one of claims 3 to 7, further comprising sealing an end cap on each end of the housing.

9. The method of any one of the preceding claims, wherein the step of treating with gamma irradiation comprises treating with a dose of gamma radiation ranging from 12.5 kGy to 175 kGy.

10. The method of claim 1, wherein the gas plasma treatment is radio frequency glow discharge.

11. The method of any one of the preceding claims, wherein the membrane comprises a polyvinyl-pyrrolidone and a polymer selected from the group consisting of polysulfone, polyethersulfone or polyarylethersulfone.

12. The method of claim 1, wherein the membrane is a flat sheet membrane.

13. The method of any one of claims 1 to 10, wherein the membrane comprises at least one hydrophilic polymer and at least one hydrophobic polymer.

14. The method of claim 13, wherein the at least one hydrophilic polymer includes polyvinylpyrrolidone, polyethylene glycol, or polysorbate.

15. The method of claim 13 or 14, wherein the at least one hydrophobic polymer includes blends of polyethersulfone, polyamide, polyaramide, polyarylethersulfone, polysulfone, polyarylsulfone, polycarbonate, polyether, polyurethane or polyetherimode, or copolymers of those polymers.

## Patentansprüche

1. Verfahren zur Montage eines Zellwachstumsmoduls, das eine synthetische Polymermembran und ein synthetisches Polymergehäuse umfasst, wobei das Verfahren Folgendes umfasst:
Behandeln der Membran mit sowohl Gammastrahlung als auch Gasplasma, um die Oberfläche der Membran zu modifizieren, um zu bewirken, dass Zellen an der Membran haften bleiben; und
Einsetzen der Membran in das Gehäuse nach dem Behandlungsschritt zur Bildung des Zellwachstumsmoduls.

2. Verfahren nach Anspruch 1, wobei die Membran eine Hohlfasermembran ist.

3. Verfahren nach Anspruch 2, wobei die Hohlfasermembran eine Vielzahl von Hohlfasern umfasst.

4. Verfahren nach Anspruch 3, ferner umfassend die Bündelung der Hohlfasern vor dem Behandlungsschritt.

5. Verfahren nach Anspruch 3, ferner umfassend die Bündelung der Hohlfasern nach dem Behandlungsschritt.

6. Verfahren nach einem der Ansprüche 3, 4 oder 5, ferner umfassend:
nach dem Einsetzungsschritt, Anhaften der gebündelten Hohlfasern an jedem Ende des Gehäuses unter Verwendung eines Einbettungsmaterials, um eine flüssigkeitsdichte Abdichtung mit dem Gehäuse zu schaffen.

7. Verfahren nach Anspruch 6, wobei das Einbettungsmaterial Polyurethan umfasst.

8. Verfahren nach einem der Ansprüche 3 bis 7, ferner umfassend die Abdichtung einer Endkappe an jedem Ende des Gehäuses.

9. Verfahren nach einem der vorstehenden Ansprüche, wobei der Schritt der Behandlung mit Gammastrahlung zusätzlich die Behandlung mit einer Gammastrahlungsdosis im Bereich von 12,5 kGy bis 175 kGy umfasst.

10. Verfahren nach Anspruch 1, wobei die Gasplasmabehandlung eine Hochfrequenz-Glimmentladung ist.

11. Verfahren nach einem der vorstehenden Ansprüche, wobei die Membran Polyvinylpyrrolidon und ein Polymer, ausgewählt aus der Gruppe bestehend aus Polysulfon, Polyethersulfon oder Polyarylethersulfon, umfasst.

12. Verfahren nach Anspruch 1, wobei die Membran eine flachlagige Membran ist.

13. Verfahren nach einem der Ansprüche 1 bis 10, wobei die Membran mindestens ein hydrophiles Polymer und mindestens ein hydrophobes Polymer umfasst.

14. Verfahren nach Anspruch 13, wobei das mindestens eine hydrophile Polymer Polyvinylpyrrolidon, Polyethylenglykol oder Polysorbat umfasst.

15. Verfahren nach Anspruch 13 oder 14, wobei das mindestens eine hydrophobe Polymer Mischungen von Polyethersulfon, Polyamid, Polyaramid, Polyarylethersulfon, Polysulfon, Polyarylsulfon, Polykarbonat, Polyether, Polyurethan oder Polyetherimod oder Copolymeren dieser Polymere umfasst.

## Revendications

1. Procédé d'assemblage d'un module de croissance cellulaire comprenant une membrane polymère synthétique et un boîtier polymère synthétique, le procédé comprenant :
le traitement de la membrane en utilisant un rayonnement gamma et du plasma gazeux pour modifier la surface de la membrane afin que les cellules adhèrent à la membrane,
l'insertion de la membrane dans le boîtier après l'étape de traitement pour former le module de croissance cellulaire.

2. Procédé selon la revendication 1, dans lequel la membrane est une membrane à fibres creuses.

3. Procédé selon la revendication 2, dans lequel la membrane à fibres creuses comprend une pluralité de fibres creuses.

4. Procédé selon la revendication 3, comprenant en outre le regroupement des fibres creuses avant l'étape de traitement.

5. Procédé selon la revendication 3, comprenant en outre le regroupement des fibres creuses après l'étape de traitement.

6. Procédé selon l'une quelconque des revendications 3, 4 ou 5, comprenant en outre :
après l'étape d'insertion, l'adhésion des fibres creuses regroupées à chaque extrémité du boîtier en utilisant un matériau d'enrobage pour créer un joint étanche aux fluides avec le boîtier.

7. Procédé selon la revendication 6, dans lequel le matériau d'enrobage comprend du polyuréthane.

8. Procédé selon l'une quelconque des revendications 3 à 7, comprenant en outre l'étanchéité d'un capuchon d'extrémité sur chaque extrémité du boîtier.

9. Procédé selon l'une quelconque des revendications précédentes, dans lequel l'étape de traitement avec un rayonnement gamma comprend en outre un traitement avec une dose de rayonnement gamma allant de 12,5 kGy à 175 kGy.

10. Procédé selon la revendication 1, dans lequel le traitement au plasma gazeux est une décharge luminescente à haute fréquence.

11. Procédé selon l'une quelconque des revendications précédentes, dans lequel la membrane comprend une polyvinyl-pyrrolidone et un polymère choisi dans le groupe constitué de polysulfone, polyéthersulfone ou polyaryléthersulfone.

12. Procédé selon la revendication 1, dans lequel la membrane est une membrane à feuille plate.

13. Procédé selon l'une quelconque des revendications 1 à 10, dans lequel la membrane comprend au moins un polymère hydrophile et au moins un polymère hydrophobe.

14. Procédé selon la revendication 13, dans lequel l'au moins un polymère hydrophile inclut de la polyvinylpyrrolidone, du polyéthylène glycol ou du polysorbate.

15. Procédé selon la revendication 13 ou 14, dans lequel le au moins un polymère hydrophobe inclut des mélanges de polyéthersulfone, polyamide, polyaramide, polyaryléthersulfone, polysulfone, polyarylsulfone, polycarbonate, polyéther, polyuréthane ou polyétherimode, ou des copolymères de ces polymères.
